# EUROPÄISCHE PATENTANMELDUNG

(11) **EP 0 609 883 A1**
(43) Veröffentlichungstag der Anmeldung: **10.08.1994**
(21) Anmeldenummer: 94101654.5
(22) Anmeldetag: 03.02.1994
(51) Int. Cl.: A61N 5/04

(54) **Flexibler Katheter**

(30) Priorität: 05.02.1993 DE 9301616 U
(71) Anmelder: W.L. Gore & Associates GmbH, D-85636 Putzbrunn (DE)
(72) Erfinder: Gronauer, Volker, D-91781 Weissenburg (DE)
(74) Vertreter: Klunker . Schmitt-Nilson . Hirsch

(57) **Zusammenfassung**

Ein flexibler Katheter (11) zum Einführen einer Mikrowellen-Energie abstrahlenden Antenne (15), insbesondere für medizinische Zwecke, weist ein Trägerrohr (23) aus massivem PTFE und auf dessen Aussenumfang eine Außenschicht (24) aus mikroporösem PTFE auf. Diese Materialwahl für den Katheter (11) führt zu guter Flexibilität, vorteilhafter Außenabpolsterung und sehr guten elektrischen Eigenschaften hinsichtlich der Mikrowellen-Energie.

## Beschreibung

Die Erfindung betrifft einen flexiblen Katheter zum Einführen einer Hochfrequenz- oder Mikrowellen-Energie abstrahlenden Antenne, insbesondere für medizinische Zwecke, mit einem Trägerrohr und einer auf dessen Außenumfang befindlichen Außenschicht aus mikroporösem Polytetrafluorethylen (PTFE).

Ein derartiger Katheter kann beispielsweise zur Mikrowellenbehandlung einer vergrößerten Prostata eingesetzt werden. Bei dieser Anwendung wird ein Katheter in die Harnröhre des zu behandelnden Mannes so weit eingeführt, daß ein am einführseitigen Ende des Katheters befindlicher, aufblasbarer Ballon in der Harnblase positioniert ist. Durch anschließendes Aufblasen des Ballons wird der Katheter in der Blase verankert. Danach wird durch ein Innenlumen des Katheters eine Mikrowellenantenne so weit eingeführt, daß sie der Prostata benachbart ist. Die Antenne ist am einführseitigen Ende eines Antennenkabels angeordnet, dessen aus dem Katheter herausragendes andere Ende mit einer Mikrowellenenergiequelle verbunden ist.

In der Nähe der Harnblase weist die Harnröhre eine relativ starke Krümmung auf. Der Katheter muß daher hohe Flexibilität mit leichter Biegbarkeit aufweisen.

Da sich die Mikrowellenantenne innerhalb des Katheters befindet, muß die Mikrowellen-Energie durch die Katheterwand hindurch zur Prostata hin abgestrahlt werden. Es sind daher nicht alle Materialien für den Katheter geeignet.

Ein herkömmlicher Katheter für die Mikrowellenbehandlung der Prostata venwendet ein mehrlumiges Katheterrohr aus Polyvinylchlorid (PVC). Ein derartiger PVC-Katheter weist zwar gute Flexibilität auf, ist aber unter elektrischen Gesichtspunkten nicht zufriedenstellend. PVC dämpt Mikrowellen zu stark, wodurch es erhitzt wird. Die Folge ist einerseits eine ungute Erweichung des Katheterschlauches und andererseits eine unerwünschte Erhitzung des den Katheter umgebenden Körpergewebes.

Die vorliegende Erfindung überwindet diese Probleme mit einem flexiblen Katheter gemäß Anspruch 1, der den Ansprüchen 2 und 3 entsprechend ausgestaltet sein kann.

Dadurch, daß bei dem erfindungsgemäßen Katheter das Trägerrohr mit massivem, d.h. nicht-porösem, Polytetrafluorethylen (PTFE) aufgebaut ist, erhält man sehr gute elektrische Eigenschaften des Katheters. PTFE ist ein für Mikrowellenabstrahlung sehr gut geeignetes Dielektrikum. Es dämpft die Mikrowellen-Energie nur sehr geringfügig. Damit wird eine Erwärmung und damit einhergehende Erweichung des Katheters infolge des Hindurchstrahlens von Mikrowellenenergie durch das Kathetermaterial weitgehend vermieden. Die geringere Erwärmung hat außerdem zur Folge, daß das Körpergewebe geringer erwärmt wird, daß man mit entsprechend reduzierter Kühlwasserkühlung auskommen kann und daß man weniger Mikrowellen-Energie in die Antenne schicken muß.

Massives PTFE hat eine geringere Flexibilität als herkömmlich verwendetes PVC. Um die erforderliche Flexibilität trotzdem sicherzustellen, macht man das Trägerrohr des erfindungsgemäßen Katheters entsprechend dünner als herkömmliche PVC-Katheterrohre. Die Restdikke des Katheterrohres erreicht man durch die Außenschicht aus mikroporösem PTFE.

Mikroporöses gerecktes PTFE ist in den US-Patentschriften 3 953 566 und 4 187 390 beschrieben. Es hat ebenfalls sehr gutes elektrisches Verhalten. Die Katheter-Außenschicht aus mikroporösem PTFE hat aufgrund der Mikroporosität gute Polstereigenschaften. Dies ist vorteilhaft, wenn der Katheter beim Bewegen in der Harnröhre auf Harnröhrenbiegungen trifft. Mikroporöses PTFE hat außerdem sehr gute Gleiteigenschaften.

Aus der DE 27 28 636 C2 ist ein für medizinische Endoskope, insbesondere Gastroskope, gedachtes Röhrchen bekannt, das einen Dreischichtenaufbau mit einer undurchlässigen Zwischenschicht, einer Aussenschicht und einer Innenschicht aufweist. Die Außenschicht und die Innenschicht bestehen aus einem porösen Polymerisat mit Fibrillenstruktur, bei dem es sich um poröses PTFE handeln kann. Die Zwischenschicht kann aus einer flexiblen Metallfolie bestehen oder aus einem dünnen flexiblen Kunststoff, beispielsweise aus FEP, PFA oder PVC. Eine flexible Metallfolie macht dieses bekannte Röhrchen für den Einsatz als Katheter zum Einführen einer Mikrowellen-Energie abstrahlenden Antenne unbrauchbar. Die Metallfolie würde die Mikrowellen-Energie vollständig abschirmen. Die für das bekannte Röhrchen vorgeschlagenen Kunststoffe, wie PVC, weisen den bereits genannten Nachteil auf, daß sie die Mikrowellen-Energie zu stark dämpfen, das heißt, einen zu großen Anteil der Mikrowellen-Energie absorbieren.

Die Außenschicht des erfindungsgemäßen Katheters kann durch ein auf das Trägerrohr aufgewickeltes Band aus mikroporösem PTFE hergestellt sein, kann als ein auf das Trägerrohr aufgebrachter Schlauch aus mikroporösem PTFE ausgebildet sein oder kann auf das Trägerrohr aufextrudiert sein, und zwar gleichzeitig oder nachträglich.

Die Erfindung wird nun anhand einer Ausführungsform näher erläutert. In den Zeichnungen zeigen:
- **Fig. 1**: in schematischer Darstellung einen Katheter zur Prostata-Mikrowellenbehandlung; und
- **Fig. 2**: einen Querschnitt durch einen erfindungsgemäßen Katheter.

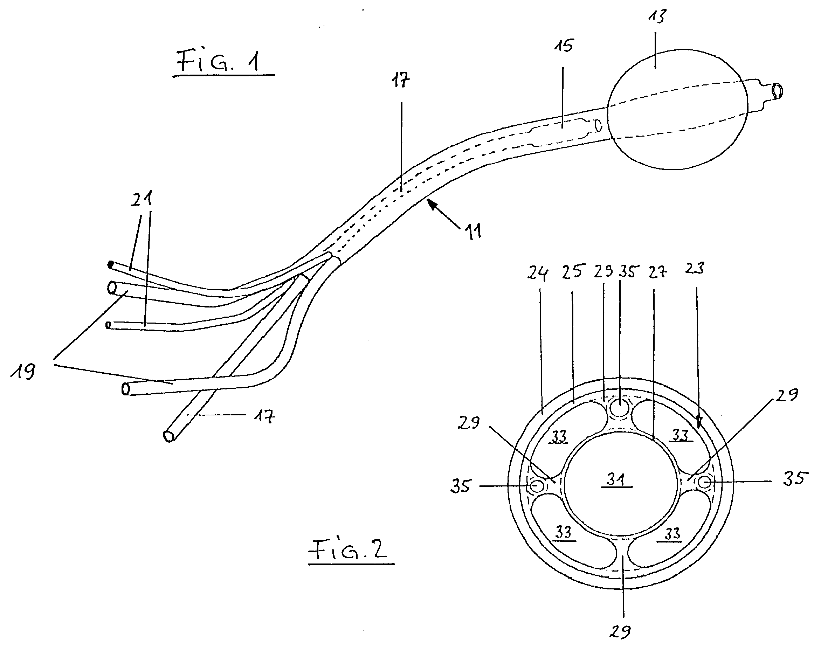

**Fig. 1** zeigt in schematischer, verkürzter Darstellung einen in den Harnleiter einführbaren Katheter 11. An dessen einführseitigem Ende befindet sich ein aufblasbarer Ballon 13, der in **Fig. 1** in aufgeblasenem Zustand gezeigt ist. In den Katheter eingeführt ist schematisch eine Mikrowellenantenne 15 am Ende einer Mikrowellenleitung 17 gezeigt. An dem der Einführseite entgegengesetzten rückwärtigen Ende des Katheters 11 sind die Mikrowellenleitung 17, zwei Kühlwasserschläuche 19 und zwei Sensorleitungen 21 aus dem Katheter 11 herausgeführt.

**Fig. 2** zeigt einen Querschnitt durch einen erfindungsgemäßen Katheter 11, der bei der bevorzugten Ausführungsform einen kreisrunden Querschnitt aufweist. Dieser Katheter 11 umfaßt ein Trägerrohr 23 aus massivem PTFE, auf dessen Außenumfang sich eine schlauchartige Außenschicht 24 aus mikroporösem PTFE befindet. Das Trägerrohr weist ein Außenrohr 25 und ein dazu konzentrisches Innenrohr 27 auf. Das Außenrohr 25 und das Innenrohr 27 sind über mehrere, bei der dargestellten Ausführungsform vier, gleichmäßig über den Umfang des Trägerrohrs 23 verteilte Radialstege 29 miteinander verbunden. Innerhalb des Innenrohrs 27 ist ein Innenlumen 31 gebildet, das zur Aufnahme der Mikrowellenantenne 15 und der Mikrowellenleitung 17 dient. Zwischen den Radialstegen 29 sind vier Außenlumen 33 gebildet, die zur Zuleitung und Ableitung von Kühlwasser verwendbar sind.

In drei der vier Radialstege 29 sind Steglumen 35 gebildet. Zwei davon dienen zur Einführung von Meß- oder Überwachungssensoren. Das in **Fig. 2** obere Steglumen 35 dient der Zuführung von Aufblasfluid, beispielsweise Luft, zum Aufblasen des Ballons 13.

Die Radialstege 29 verleihen dem Außenrohr 25 und damit dem gesamten Katheter 11 eine gute mechanische Stabilität gegen Einknicken und damit Abklemmen einzelner Lumen. Die Außenschicht 24 aus mikroporösem PTFE weist nicht nur gute Polstereigenschaften, Gleiteigenschaften und elektrische Eigenschaften auf, sondern fängt bei starken Abbiegungen des Katheters 11 Stauchwirkungen auf, so daß Faltenbildungen am Außenumfang des Katheters 11 vermieden werden.

## Patentansprüche

1. Flexibler Katheter (11) zum Einführen einer Hochfrequenz- oder Mikrowellen-Energie abstrahlenden Antenne (15), insbesondere für medizinische Zwecke, mit einem Trägerrohr (23) und einer auf dessen Außenumfang befindlichen Außenschicht (24) aus mikroporösem Polytetrafluorethylen (PTFE),
**dadurch gekennzeichnet,**
daß das Trägerrohr (23) mit massivem PTFE aufgebaut ist.

2. Katheter nach Anspruch 1,
**dadurch gekennzeichnet,**
daß das Trägerrohr (23) eine Doppelwandstruktur mit einem Außenrohr (25) und einem dazu konzentrischen Innenrohr (27) aufweist, die mittels einer Mehrzahl vorzugsweise gleichmäßig über den Umfang verteilter Radialstege (29) miteinander verbunden sind, so daß innerhalb des Innenrohres (27) ein Innenlumen (31) zum Einführen der Antenne (15) und zwischen Innenrohr (27) und Außenrohr (25) mehrere Außenlumen (33), insbesondere zum Transport von Fluiden und/oder zum Einführen von Meß- oder Überwachungsvorrichtungen, gebildet sind.

3. Katheter nach Anspruch 2,
**dadurch gekennzeichnet,**
daß im Querschnitt mindestens eines der Radialstege (29) mindestens ein Steglumen (35) gebildet ist.
